# EUROPEAN PATENT SPECIFICATION

(11) **EP 2 338 046 B1**
(45) Date of publication and mention of the grant of the patent: **21.12.2016**
(21) Application number: 09782935.2
(22) Date of filing: 11.09.2009
(51) Int. Cl.: G01N 27/64, H01J 49/00, G01N 33/22

(54) **METHOD AND DEVICE FOR THE DETECTION OF ANIONS**
VERFAHREN UND VORRICHTUNG ZUM NACHWEIS VON ANIONEN
PROCÉDÉ ET DISPOSITIF POUR LA DÉTECTION D ANIONS

(30) Priority: 12.09.2008 DE 102008042055; 16.09.2008 EP 08164439
(43) Date of publication of application: 29.06.2011
(73) Proprietor: Universität Potsdam, 14469 Potsdam (DE)
(72) Inventor: BEITZ, Toralf, 14476 Golm (DE); LAUDIEN, Robert, 14471 Potsdam (DE); LÖHMANNSRÖBEN, Hans-Gerd, 14476 Golm (DE)
(74) Representative: Schubert, Klemens
(86) International application number: PCT/EP2009/061830
(87) International publication number: WO 2010/029165

(56) References cited:
- WO-A-2007/014019
- US-A- 3 697 748
- US-A1- 2007 085 001
- R FROMHERZ, G GANTEFÖR AND A SHVARTSBURG: "Isomer-Resolved Ion Spectroscopy" PHYSICAL REVIEW LETTERS, vol. 89, no. 9, 6 August 2002 (2002-08-06), pages 083001-1-083001-4, XP002555404

## Description

The present invention relates to a method and a device for the detection of anions.

Ion mobility (IM) spectrometry is already established as a sensor technique for the trace detection of volatile organic compounds, chemical warfare agents, illicit drugs, toxic industrial compounds and explosives (Eiceman, G. A.; Karpas, Z. Ion Mobility Spectrometry, 2nd Edition, CRC Press, Boca Raton, 2005). The most important advantages of commercial IM spectrometry are the short measurement time, enabling almost real-time response, and the high sensitivity in the low ppb range. IM spectrometry as analytical method is commercially applied, e.g., in walkthrough portals for the trace detection of explosives or explosive related compounds (ERC) at airports. In industrial applications, the determination of nitroaromatic compounds (NAC), such as TNT, in commercial IM spectrometers is based on the formation of anions by reaction with chloride ions (Equation 1) (K. A. Daum, D. A. Atkinson, R. G. Ewing Talanta 2001, 55, 491-500):

**NAC + Cl⁻ → [NAC-H]⁻ + HCl** **(1)**

Here, the chloride ions are formed by the reaction of thermal electrons with a chlorine-containing dopant, like dichloromethane. The selectivity of the detection is based on the combination of the acidity of the NAC, of the positive electron affinity (EA) of the [NAC-H] radicals, and of the characteristic ion mobility of the [NAC-H]⁻ anions. The alternative NAC detection as protonated cations via proton transfer is less selective as the NAC proton affinity (PA) is too low.

The selectivity of commercially applied detection of nitroaromatic ERC is limited by interfering substances, which also have a high acidity and a positive EA, and show a similar ion mobility (B. Kanu, C. Wuc, H. H. Hill Anal. Chim. Acta 2008, 610, 125-134; L. M. Matz, P. S. Tornatore, H. H. Hill Talanta 2001, 54, 171-179; K. A. Daum, D. A. Atkinson, R. G. Ewing, W. B. Knighton, E. P. Grimsrud Talanta 2001, 54, 299-306). Among these substances are nitrated and halogenated phenols, which are ubiquitous in the environment. These compounds can lead to false-positive alarms and corresponding psychological and economic damages. A possible improvement for increasing selectivity in IM spectrometry is the addition of a GC column or, alternatively, of selective sorbents. However, this is clearly on the cost of real-time response capability. Therefore, it is of scientific and of economic interest to find a real-time criterion for elucidating whether signals in the IM. spectrum can be attributed to explosives, like TNT, or to interfering compounds. Such a criterion could be derived from in situ spectroscopic characterisation of the ion clouds in the IM spectrometer. Here, low ion number densities are encountered, so that high detection sensitivity, as e.g. provided by laser induced fluorescence (LIF) or charge detection, is required. The more sensitive detection of charges is among others based on their efficient electric-field-guidance towards the detector, whereas LIF photons are only captured to a small fraction. Furthermore, charge detection in an IM spectrometer is more economic as no additional photodetectors are necessary. Promising charge detection techniques are photodissociation and photodetachment (PD) spectroscopy (D. M. Wetzel, J. I. Brauman Chem. Rev. 1987, 87, 607-622). In MS, photoelectron-PD-spectroscopy is successfully applied, by which the kinetic energy of photodetached electrons and thus the energy levels of the investigated compounds are determined (M. Tschurl, C. Ueberfluss, U. Boesl Chem. Phys. Letters 2007, 439, 23-28; S. M. Seehan, B. F. Parsons, T. A. Yen, M. R. Furlanetto, D. M. Neumark J. Chem. Phys. 2008, 128, 174301). This kind of energy-resolved spectroscopy is not feasible under atmospheric pressure as after electron-bath gas collisions the information on the initial energy is lost. Therefore, only the integral electron yield as function of the wavelength of the anions can be measured giving wavelength-dependent PD cross sections. Grimsrud et al. have measured PD spectra of some NAC with a broad-band xenon lamp radiation showing a strong dependence of the PD cross sections on the wavelength (R. S. Mock, E. P. Grimsrud J. Am. Chem. Soc. 1989, 111, 2861-2870).

IM spectrometry allows for fast real-time on-site analyses of chemical substances with high sensitivity down to the ppb range. This technique is based on the measurement of the migration velocities of ionized molecules countercurrent with a flow of gas, the so called drift gas, through an electricial field. In IM spectrometry explosives based on nitro aromatics like 2,4,6-trinitrotoluene (TNT) are detected as anions. The selectivity of the formation of anions is due to the positive electron affinity (in nitrogen) and the relatively high acidity (in oxygen), as well as the positive electron affinity of the [NAC-H] radicals, respectively, of the nitro aromatic compounds depending on the drift gas used. Depending on the drift gas used and on the temperature the anions formed have a characteristic ion mobility and drift time, respectively. The selectivity of the detection of the nitro aromatics in IM-Spectroscopy is based on the combination of specific ionisation and characteristic ion mobility. In the literature a number of compounds is known that are responsible for the false positive detection of the nitro aromatics, i.e. these compounds also form anions and are detected at a similar drift time. For example, at airport terminals such false alarms can be associated with large costs, cause unnecessary inconveniences to the passengers and can lead to a less careful handling of the security equipment.

Until now a gas chromatography column is installed upstream to solve this problem. But this solution leads to a loss of real-time capability of the method, i.e. the response times change from the lower seconds range to response times within the minutes range. In areas where there is a need for a high throughput of analytes within a short period of time this method can only be utilized selectively. A general systematic screening is no longer possible.

It is therefore an aim of the present invention to overcome the disadvantages that are known from the state of the art.

The problem is solved by a method according to claim 1 of the present invention. The problem is also solved by a device for carrying out the method according to the invention. The problem is also solved by the use of the method and/or the device according to the present invention for the detection of anions in samples.

According to the invention the problem is solved by providing a method for the detection of anions by combining ion mobility spectrometry (IM) at atmospheric pressure with photo detachment spectroscopy (PD), wherein the following steps are performed:
introducing a sample gas into a drift tube;
ionizing the sample molecules to be detected in sample gas;
applying an electrical field on the ionized sample molecules, whereby the ionized sample molecules are mobilized in the drift tube in direction to a detector;
applying photo detachment energy on the ionized gas sample in spatial proximity to the said detector, whereby photo detached electrons are generated;
detecting simultaneously the ions of the sample molecules and the photo detached electrons; and comparing the intensity of the ion peaks and the electron peaks.

The method according to the invention is further characterized in that the sample molecules are ionized by means of an electromagnetic energy source or by means of a laser energy source or by means of a radioactive energy source or by means of a negative electrospray source or by means of a corona discharge source (APCI) or by XUV photon energy or by the REMPI technique or by a combination of the said energy sources or techniques. A person skilled in the art may use other energy sources that are suitable to produce ions that can be detected by IM.

According to the invention it is preferred that the sample gas is ionized by means of a electromagnetic energy source in a X-range between 100 nm and 500 nm.

According to the invention the photo detachment energy is applied by means of a laser energy source or other light sources, wherein the λ-range is between 200 nm and 1400 nm. A person skilled in the art may use other energy or light sources that are suitable to produce ions that can be detected by IM.

According to the invention it is preferred that the photo detachment energy is applied by means of a laser energy source simultaneously or consecutively at two or more different wavelengths in λ-ranges between 200 nm and 1400 nm.

It is especially preferred according to the invention that the ionization energy sources and/or the photo detachment energy sources are pulsed and that the pulses are timely shifted.

The method according to the invention is further characterized in that the photo detachment cross sections of the detected ions are determined and compared.

It is also preferred that the ions belonging to one peak are additionally to the ion mobility characterized by the photo detachment cross section. This means that the method according to the invention is characterized in that the ions belonging to one peak can be characterised additionally to the ion mobility by the PD cross section. The combination of PD cross section and drift time increases the selectivity of detection and solves the problem of false positive detection.

The method according to the invention is therefore characterized in that at complete PD the height of the electron peak is significantly higher than the anion peak leading to an increase of the sensitivity.

It is also preferred that the peak purity is determined by the determination of the photo detachment of ions in the rising and in the falling edges of the respective peak. This means in other words that the method according to the invention is characterized in that the determination of PD of ions in the rising and the falling edges of the peak allow the determination of the peak purity.

Furthermore, the method according to the invention is characterized in that in addition to electrons photo dissociation products can occur. Their drift times give information about their identity and their peak area about their photodissoziation cross section.

Another object of the present invention is a device 1 for carrying out a method according to one of the preceding claims, whereby the device 1 comprises:
a drift tube 2, being divided into a reaction region 4 and a drift region 5;
a sample inlet 6, located in the reaction region 4;
a drift gas inlet 7, located in the drift region 5;
a detector plate 3, located within the drift region 5 at one end of the drift tube 2; and
means for providing ionizing energy or at least one pair of windows 8 being placed in the drift tube 2 within the reaction region 4;
characterized in that
at least one additional pair of windows 9 is present in the drift tube 2 within the drift region 5 of the drift tube 2.

A device according to the invention is preferred, wherein one of the windows 8 and/or one of the windows 9 is replaced by a glass-fiber optics inlet.

A device is also preferred, wherein there is a pair of windows 8 placed on opposite ends of a diameter of the drift tube 2 in the reaction region 4 and that there is a pair of additional windows 9 placed on opposite ends of a diameter of the drift tube 2 in the drift region 5 and that the windows 8 and the additional windows 9 are glass windows and/or quartz glass windows. It is obvious for the skilled artesian to use quartz glass windows for UV light and normal glass windows for visible light.

A device according to the invention is also preferred, wherein the reaction region 4 and the drift region 5 are separated from each other by at least one shutter or ion gate 10, 10a, whereby a shutter 10 is placed in proximity to the sample inlet 6 in order to delimit the reaction region and/or an ion gate 10a is placed in upstream direction of the ion flow in proximity to the additional windows 9.

A device according to the invention can be produced, for example, by using a drift tube for IM according to the state of the art and applying additional windows or inlets to that drift tube.

A further object of the present invention is the use of a method according to the invention as described herein for the detection of volatile organic compounds, chemical warfare agents, illicit drugs, toxic industrial compounds and explosives.

Just another object of the present invention is the use of a device according to the invention as described herein for the detection of volatile organic compounds, chemical warfare agents, illicit drugs, toxic industrial compounds and explosives.

The present invention is now described in more detail with reference to the attached figures and the following description and examples.

### Short description of the figures:

In the figures the term "rel. u." stands for "relative units".
Figure 1 shows the principle of the method according to the present invention;
Figure 1a shows a first embodiment of a device according to the invention;
Figure 1b shows a second embodiment of a device according to the invention;
Figure 2a shows an IM spectrum of 2,4-DNT obtained from laser ionisation (X = 266 nm) of toluene (lower trace) and a PD-IM spectrum (upper trace) after photo detachment (λ = 532 nm, first peak - detached electrons, second peak - dissociation products, possibly NO₂⁻, third peak - depleted anions);
Figure 2b shows a tomography experiment: Scanning of the anion cloud with the PD laser by variation of the delay time Δt with respect to the ionisation laser. PD-IM spectra of 2,4-DNT (signals around 2.3 ms are due to superimposed PD electron peaks, signal at 3.5 ms is anion peak, the signal in the 1 - 2 ms range is an experimental artefact);
Figure 3a shows an IM spectrum of the two-component mixture of 1,3-dinitrobenzene and TNT (the signal in the 1 - 2 ms range is an experimental artefact);
Figure 3b shows PD-IM spectra of 1,3-dinitrobenzene (upper trace) and TNT (lower trace);
Figure 4 shows PD-IM spectra of [TNT], variation of the PD laser energy between 0.5 - 3 mJ (signals at 2.3 and 3.5 ms are PD electron and anion peaks, respectively, signal in the 1 - 2 ms range is an experimental artefact). The signal obtained with 0.5 mJ (middle trace) displays a signal-to-noise ratio > 15;
Figure 5a shows IM (lower trace) and PD-IM (upper trace) spectra of 2,4-DNT;
Figure 5b shows IM (lower trace) and PD-IM (upper trace) spectra of 2,4-DNP;
Figure 6a shows IM (lower trace) and PD-IM spectra of TNT, variation of PD laser energy between 0.5 mJ (central trace) and 8 mJ (upper trace). The first high signal (app. 1.7 ms) can be attributed to chloride ions, the two other large signals to PD electrons (2.2 ms) and to [M-H]⁻ anions (3.4 ms); and
Figure 6b shows IM (lower trace) and PD-IM (upper trace) spectra of TNT (drift gas nitrogen, 4 mJ PD laser energy), wherein the inset shows the electron peak.

### Detailed description of the invention

A solution of the problem with false positive alarms in real time could be the spectroscopic characterization of the anions. But the very low number of ions compared with the concentration of neutral particles does not allow the use of established optical spectroscopic methods. At present a sensitive spectroscopic detection can only be based on a change in the structure of the anions after the interaction with light (laser beam), that does not reduce the number of detected charges and will be visible in the IM spectrum. Known spectroscopic methods that meet these criteria are from the area of mass spectrometry (MS) photo dissociation (PDi) spectroscopy (for cations and anions) and photodetachment (PD) spectroscopy (for anions). But both methods have not yet been applied in In spectrometry. In MS PDi spectroscopy is used for structure characterization and PD spectroscopy is applied in the context of basic investigations. But even in MS analytical applications are not yet known.

The present invention is based on the utilization of differences in photo detachment (PD) cross sections of the anions of explosives and the interfering molecules, respectively, that produce the false positive detection. Upon interaction with laser light of adequate intensity and wavelength, depending on the PD cross section, a different number of electrons can be detached from the anion cloud, which produce an additional characteristic peak within the IM spectrum.

The comparison of the intensities of electron and anion peak allows for conclusions concerning the PD cross section and therefore allows for differentiating between explosives molecules and interfering molecules. Generally only a few PD cross sections (PDQS) are published in the literature and in particular no information concerning PDQS of explosives and known interfering molecules, respectively, are available. Relative PDQS determined by the inventors show differences in the area of more than one order of magnitude between nitrated toluenes and nitrated phenols and halogenated phenols, respectively, at a wave length of λ = 532 nm. The differences in the PDQS of the individual compounds are sufficient for an analytical discrimination and allow for an adequate characterization of the anions. Furthermore the PDQS depend on the wavelength. With a known interfering compound in a two colour experiment its proportion can be quantified.

Additionally with alternative wavelengths characteristic dissociation products are observed.

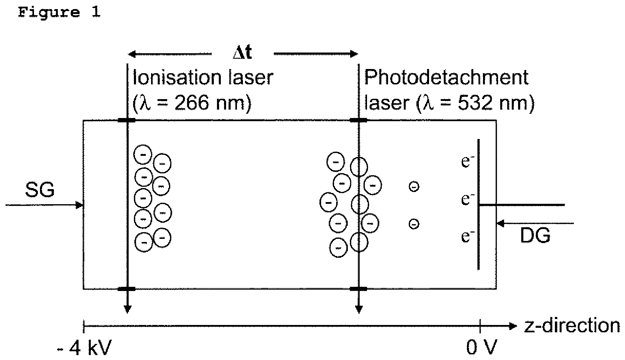

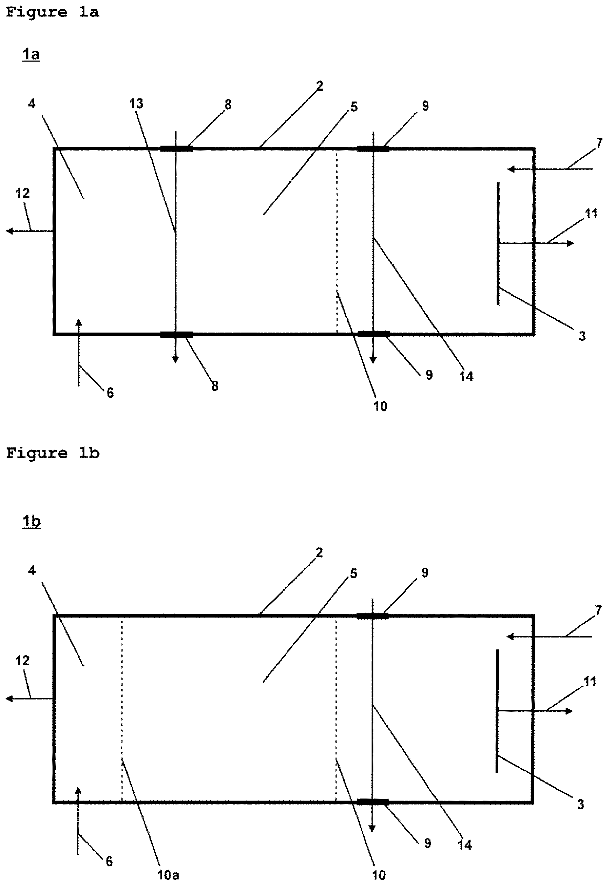

The realization of the invention is now based on the formation of the ions and the separation of the produced ions in the first part 4 of the drift tube 2 (Fig. 1a,1b).

As shown in Fig. 1a and 1b, this first part 4 is followed by an area with two laser windows 9 arranged opposite to one another. The electrons detached by this PD process are separated from the remaining anions in a further unit 5 of the drift tube 2 and are detected on the Faraday plate 3. The detection of the electrons occurs because of their high mobility immediately after the laser pulse. By this the length of the second part 5 of the drift tube 2 can be kept very short. By variation of the time shift of the pulses for ion formation (laser ionisation) or of the ion gate 10a (formation of ion pulse; radioactive source) and of the PD-laser pulse the complete spectrum can be scanned and the generated peaks can be characterized.

In order to avoid a possible overlap of the electron peaks with the anion peaks of further ingredients of the analyte mixture, the peaks to be investigated can be isolated by an ion gate 10.

An easier way to avoid an overlap of the electron and anions peaks is the variation of the drift voltage.

The developed electron peak has a considerable lower full width at half maximum compared to the anion peak. Since the peak area in an IM spectrum is proportional to the detected charge and the charge is essentially retained (apart from losses by diffusion) with a complete PD the signal-to-noise ratio and thus the detection limit of explosives can be additionally improved.

Compared to conventional IM spectrometry the present invention leads to both an improvement of the selectivity and of the detection limit.

The inventors have investigated the interaction of laser radiation with anions under release of a negative charge (the photo detached electron) from the anions (Equation 2):

**NAC⁻ + hv → NAC + e⁻** **(2)**

For the first time, IM spectrometry at atmospheric pressure is combined with PD spectroscopy. The combination of both methods is described. Relative PD cross sections of several nitrotoluenes and nitrophenols at a laser radiation of λ = 532 nm are determined, and their potential for an unambiguous characterisation of the nitrotoluenes is shown.

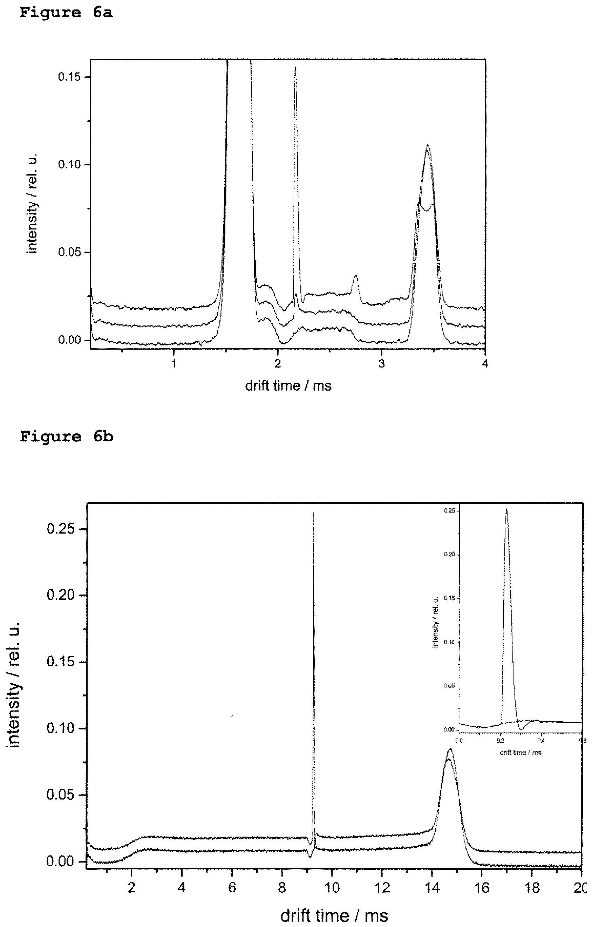

In commercial IM spectrometers, the ERC react with chloride ions under formation of [M-H]⁻ anions which are finally detected. In order to prove the concept of the present invention also for these species PD experiments were performed with the [M-H]⁻ anions of 2,4-DNT and TNT. The respective PD-IM spectra for TNT are shown in Figure 6a. The first high signal can be attributed to the chloride ions, the two other signals to the photo detached electrons and to the [M-H]⁻ anions. The obtained PD cross sections are of the same order than the ones of the [M]⁻ anions. Thus, the above described method should also be applicable for the [M-H]⁻ anions. Figure 6b finally shows a PD-IM spectrum recorded in nitrogen as drift gas that is more convenient for practical applications than helium. Compared to the investigations in helium the relation of peak height and peak area is appreciably larger. This improves the detection limit of the PD method.

The combination of IM spectrometry and PD spectroscopy allows a real-time characterisation of the anion peaks in the IM spectrum in order to differentiate between nitro aromatics and interfering substances and, thus, to reduce false positive detections of nitro aromatics. The electrons detached by the PD laser at λ = 532 nm are detected in the same IM spectrum as the anions. The relation of the peak areas of detached electrons to the respective anions is proportional to the PD cross section at constant laser energy. The PD efficiencies of the anions are shown for a number of nitro aromatics like 2,4-dinitrotoluene and TNT and of potentially interfering substances like 2,4-dinitrophenol and pentachlorophenol. The PD cross sections of the nitro aromatics are around one order of magnitude higher than the ones of the interfering substances and, thus, can unambiguously be distinguished.

The method and the device according to the present invention solve the problem of false positive detection of ions and provide a higher selectivity and greater sensitivity.

### Examples

### Photodetachment spectroscopy.

For these experiments the IM spectrometer was modified in the following way: Along the drift region 5 additional quartz glass windows 9 were inserted at a position of around 2/3 in direction of the Faraday plate 3 in order to enable the entrance of laser light for detachment. These windows 9 were placed perpendicular to the windows 8 of the ionisation region 4. As ionisation source a Nd:YG laser (continuum, Santa Clara, U.S.A., Minilite II with second, third and fourth harmonic generation) was used. At 266 nm, laser beam size and energy were also typically 1 mm and 100 µJ in 8 ns laser pulses. For the detachment of electrons from the anions the second harmonic generation of the Nd:YAG laser PRO 190-10 from Spectra Physics (532 nm) was used. The laser beam entered the detachment region either through a pin hole with 1 - 5 mm diameter or through a slit with 0.5 - 5 mm width, the laser energy varied in the range 0.5 - 100 mJ in 8 ns laser pulses. The time of detachment was delayed with respect to the time of ionisation by a digital delay/pulse generator (Scientific Instruments GmbH, Gilching, Germany, DG535). The delay time Δt was around 2/3 of the drift time of the investigated anions.

A schematic description of the PD experiment is shown in Figure 1. The principle of PD-IM spectrometry is summarized as follows:
Formation of NAC anions by laser ionisation (λ = 266 nm) and following IMR, drift of anions through drift tube, application of photo detachment laser (A = 532 nm) after delay time Δt between ionisation laser and PD laser, neutralisation of anions, formation of PD electrons (e⁻) and dissociation products (SG: sample gas, DG: drift gas). Variation of Δt allows tomographic mapping of ion cloud (see below).

### Characterization of nitro aromatic anions by photo detachment.

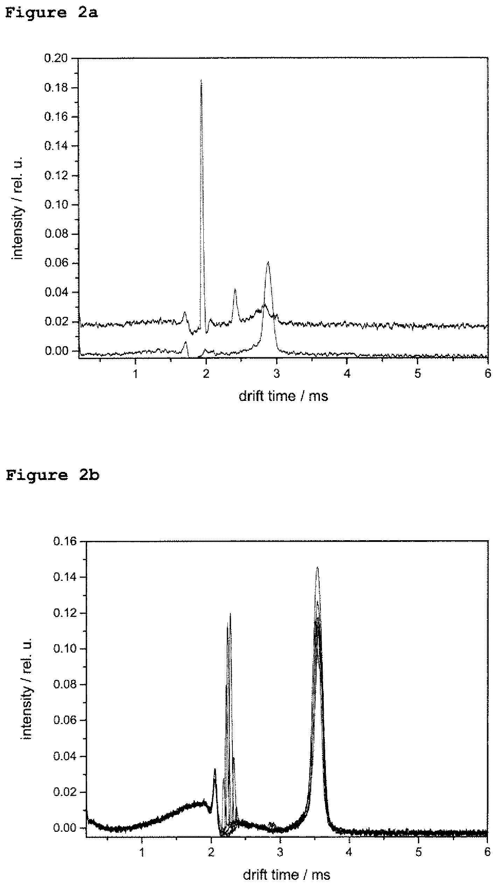

Electron PD resulted in strong peaks superimposed with the IM spectra of the corresponding anions. As an example, the PD-IM spectrum of 2,4-DNT is shown in Figure 2a. The complete information about PD is obtained by comparing the IM spectra recorded with and without detachment laser. Due to the high mobility of the electrons, they are detected almost instantaneous with respect to the PD laser pulse and the PD peak is very narrow compared to the anion peak. The width of the PD peak is caused by the rise time of the amplifier (t > 40 µs). Importantly, electron PD leads to a significant increase of the detection sensitivity due to the high narrow electron peak. Faster amplifiers with appreciably lower rise time will further increase sensitivity. In complex IM spectra, e.g. resulting from multi-component mixtures, the overlap of the electron peak with other anion peaks will complicate the analysis. This problem can be solved by the integration of an ion gate 10 into the drift tube 2 separating anion and electron peaks. This leads to considerable simplified IM spectra. The ion gate 10 should be integrated directly in front of the detachment region 5, enabling an optimal pre-separation of the anion peaks using the drift tube. For electron detection the length of the drift tube after the detachment region can be relatively short if inert drift gases are used. In air, by attachment of electrons to the oxygen, O₂⁻ ions are formed, the separation of which demands longer drift distances.

The laser PD method allows the three-dimensional, tomographic mapping of an ion cloud within an IM spectrometer. The inventors believe that this has not been demonstrated before. Whereas so far, from ion arrival time at the Faraday plate, information about the ion cloud was only obtained in direction of the electric field (z-axis), now the extension and ion density distribution of the ion cloud can be determined in both directions perpendicular to the electric field (x- and γ-axis). Thus, besides the longitudinal diffusion coefficients in electric field direction also the transversal diffusion coefficients for perpendicular movement can be determined. In Figure 2b the tomographic scan of the ion cloud of 2,4-DNT anions in the z-direction by variation of the time delay between the pulses of ionisation and PD laser is shown. The envelope of the electron peaks characterises the extension and ion density distribution of the anion cloud after a distance of 2/3 of the drift tube.

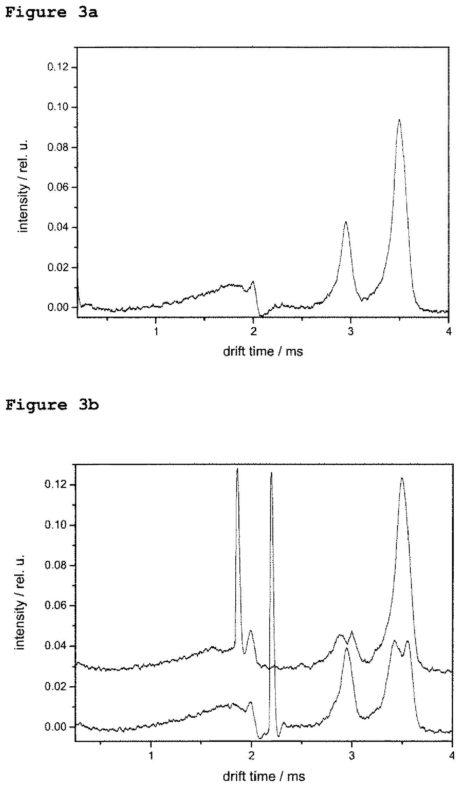

In multi-component mixtures different anion peaks can be characterised sequentially by varying the time delay between ionisation and PD laser. As example, in Figure 3a the IM spectrum of the two-component mixture of 1,3-dinitrobenzene and TNT is shown. The separation of the signals derived from the two anions is clearly discernible. Figure 3b shows the superposition of the PD-IM spectra of both anions. For both anions the characteristic relative PD cross sections can be determined. Apart from the two strong PD electron signals another interesting feature is evident in Figure 3b: In analogy to spatial hole burning, the PD process leads to depletion of the anionic species which leads to the distinct minima in the corresponding signals.

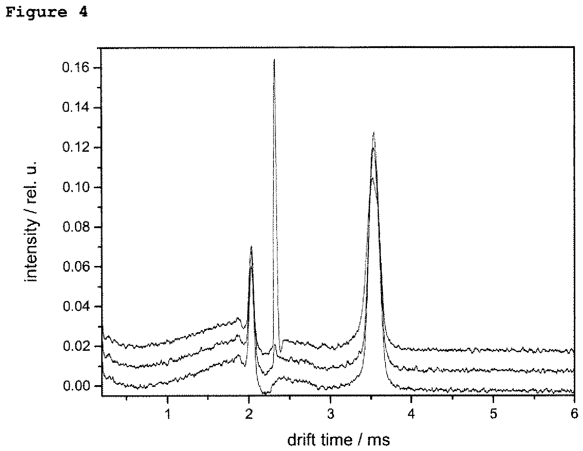

Investigations on the dependence of the PD yield on the laser intensity are necessary both for basic determinations of PD cross sections and for the evaluation of required pulse energies of the PD laser with respect to analytical applications. Under economic aspects, low-intensity lasers are of interest. This evaluation was performed for three NAC, namely 1,3-dinitrobenzene (DNB), 2,4-DNT and TNT. In Figure 4 the PD-IM spectra for TNT in dependence on the laser intensity are shown. In the PD-IM spectra a direct correlation between the peak area of the photodetached electrons and the laser intensity is seen. For PD at X = 532 nm with 8 ns laser pulses, the smallest laser intensities for safe PD electron detection (signal-to-noise ration of ca. 3) were below 0.2 mJ for 2,4-DNT, below 0.3 mJ for 1,3-DNB and below 0.1 mJ for TNT. Thus, commercially available and inexpensive DPSS lasers are suitable for analytical applications of PD-IM spectrometry.

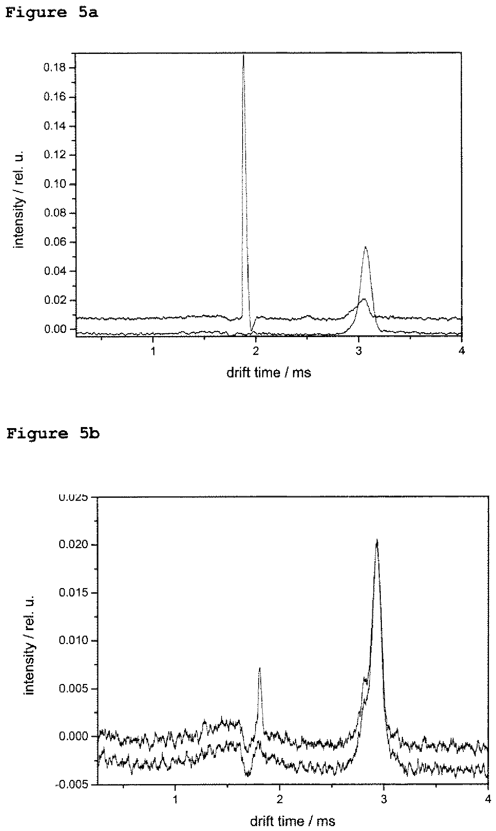

Integrating of PD spectroscopy into the IM spectrometry has great potential for the real-time differentiation between ERNC and interfering substances which are ubiquitous in the environment. Therefore, the PD-IM spectra of several nitrated toluenes as ERNC, and of nitrated and halogenated phenols as interfering compounds have been recorded. In the literature information on electron affinities and PD spectra are available only for nitrobenzene, 2,4-DNT and 2,6-DNT (Mock et al.). In Figures 5a and 5b, the IM and PD-IM spectra of 2,4-DNT, as typical ERNC, and of 2,4-dinitrophenol (DNP, interfering substance) are shown. With identical PD laser intensities, the peak area ratios of the PD electrons to parent anions differ by app. one order of magnitude. This reflects significantly different PD cross sections and enables a reliable differentiation of both compounds.

In commercial IM spectrometers, the ERNC react with chloride ions under formation of [M-H]⁻ anions which are finally detected. In order to validate the concept of the invention also for these species PD experiments were performed with the [M-H]⁻ anions of 2,4-DNT and TNT. The respective PD-IM spectra for TNT are shown in Figure 6a.

The first high signal (at app. 1.7 ms) can be attributed to chloride ions, the two other large signals to PD electrons and to [M-H]⁻ anions. It is estimated that the obtained PD cross sections are of the same order than the ones of the [M]⁻ anions. Thus, the PD-IM method is also applicable for characterization and detection of [M-H]⁻ anions. Finally, Figure 6b shows IM and PD-IM spectra of TNT recorded in nitrogen as drift gas. In comparison to helium the PD electron peak is significantly more pronounced which improves the detection limit of the PD method considerably.

For on-site applications, like on airports, the device is constructed in a compact manner. Therefore, the device is easy to transport and can be used in different places without the need for time consuming assembly procedures.

The sampling of surfaces, either of a part of a person's body or of any other surface, can be accomplished as it is known in the state of the art.

Sampling is performed by bringing an adequate adsorption material, like a fibrous web, into contact with the surface to be scanned. In this way particles and dust are removed from the surface to the adsorption material. Additionally the adsorption material can comprise an appropriate solvent to release compacted substances from the surface to be scanned.

Bringing into contact can be accomplished by wiping. In this case the sampling is called "wipe test".

Furthermore it is known in the state of the art to sample substances from surfaces by means of the application of negative or positive pressure. According to the pressure applied particles and dust are withdrawn from the surface by suction or are blown off the surface. In both cases the device comprises means for collecting the particles and the dust, like filter materials.

### List of reference signs

- 1: detection device
- 2: drift tube
- 3: detector / detector plate / Faraday plate
- 4: reaction region
- 5: drift region
- 6: sample inlet
- 7: drift gas inlet
- 8: window
- 9: additional window
- 10, 10a: shutter / ion gate
- 11: to amplifier (not shown)
- 12: gas outlet
- 13: ionisation energy source
- 14: photo detachment laser

## Claims

1. A method for the detection of anions by combining ion mobility spectrometry at atmospheric pressure with photo detachment spectroscopy, wherein the following steps are performed:
introducing a sample gas into a drift tube;
ionizing the sample molecules to be detected in sample gas;
applying an electrical field on the ionized sample molecules, whereby the ionized sample molecules are mobilized in the drift tube in direction to a detector;
applying photo detachment energy on the ionized gas sample in spatial proximity to the said detector, whereby photo detached electrons are generated;
detecting simultaneously the ions of the sample molecules and the photo detached electrons; and comparing the intensity of the ion peaks and the electron peaks.

2. The method according to claim 1, **characterized in that** the sample molecules are ionized by means of an electromagnetic energy source or by means of a laser energy source or by means of a radioactive energy source or by means of a negative electrospray source or by means of a corona discharge source (APCI) or by XUV photon energy or by the REMPI technique or by a combination of the said energy sources or techniques.

3. The method according to claim 2, **characterized in that** the sample gas is ionized by means of an electromagnetic energy source in a λ-range between 100 nm and 500 nm.

4. The method according to one of the preceding claims, **characterized in that** the photo detachment energy is applied by means of a laser energy source or other light sources, wherein the λ-range is between 200 nm and 1400 nm.

5. The method according to one of the preceding claims, **characterized in that** the photo detachment energy is applied by means of a laser energy source simultaneously or consecutively at two or more different wavelengths in λ-ranges between 200 nm and 1400 nm.

6. The method according to one of the preceding claims, **characterized in that** the ionization energy sources and/or the photo detachment energy sources are pulsed and that the pulses are timely shifted.

7. The method according to one of the preceding claims, **characterized in that** the photo detachment cross sections of the detected ions are determined and compared.

8. The method according to one of the preceding claims, **characterized in that** the ions belonging to one peak are additionally to the ion mobility **characterized by** the photo detachment cross section.

9. The method according to one of the preceding claims, **characterized in that** the peak purity is determined by the determination of the photo detachment of ions in the rising and in the falling edges of the respective peak.

10. A device (1) for carrying out a method according to one of the preceding claims, whereby the device (1) comprises:
a drift tube (2), being divided into a reaction region (4) and a drift region (5);
a sample inlet (6), located in the reaction region (4) ;
a drift gas inlet (7), located in the drift region (5) ;
a detector plate (3), located within the drift region (5) at one end of the drift tube (2); and
means for providing ionizing energy or at least one pair of windows (8) being placed in the drift tube (2) within the reaction region (4);
**characterized in that**
at least one additional pair of windows (9) is present in the drift tube (2) within the drift region (5) of the drift tube (2).

11. The device according to claim 10, **characterized in that** one of the windows (8) and/or one of the windows (9) is replaced by a glass-fiber optics inlet.

12. The device according to claim 10, **characterized in that** there is a pair of windows (8) placed on opposite ends of a diameter of the drift tube (2) in the reaction region (4) and that there is a pair of additional windows (9) placed on opposite ends of a diameter of the drift tube (2) in the drift region (5) and that the windows (8) and the additional windows (9) are glass windows and/or quartz glass windows.

13. The device according to claim 10, **characterized in that** the reaction region (4) and the drift region (5) are separated from each other by at least one shutter or ion gate (10, 10a), whereby a shutter (10) is placed in proximity to the sample inlet (6) in order to delimit the reaction region and/or an ion gate (10a) is placed in upstream direction of the ion flow in proximity to the additional windows (9).

14. Use of a method according to any one of the claims 1 to 9 for the detection of volatile organic compounds, chemical warfare agents, illicit drugs, toxic industrial compounds and explosives.

15. Use of a device according to any one of the claims 10 to 13 for the detection of volatile organic compounds, chemical warfare agents, illicit drugs, toxic industrial compounds and explosives.

## Patentansprüche

1. Verfahren zur Detektion von Anionen durch Kombinieren von Ionenmobilitätsspektrometrie bei atmosphärischem Druck mit Photodetachmentspektroskopie, worin die folgenden Schritte durchgeführt werden:
Einführen eines Probengases in eine Driftröhre;
Ionisieren der im Probengas zu detektierenden Probenmoleküle;
Anlegen eines elektrischen Feldes an die ionisierten Probenmoleküle, wodurch die ionisierten Probenmoleküle in der Driftröhre in Richtung auf einen Detektor mobilisiert werden;
Anwenden von Photodetachmentenergie auf die ionisierte Gasprobe in räumlicher Nähe zu dem genannten Detektor, wodurch photodetachte Elektronen erzeugt werden;
gleichzeitiges Detektieren der Ionen der Probenmoleküle und der photodetachten Elektronen; und Vergleichen der Intensität der Ionenpeaks und der Elektronenpeaks.

2. Verfahren gemäß Anspruch 1, **dadurch gekennzeichnet, dass** die Probenmoleküle mittels einer elektromagnetischen Energiequelle oder mittels einer Laserenergiequelle oder mittels einer radioaktiven Energiequelle oder mittels einer negativen Elektrosprayquelle oder mittels einer Koronaentladungsquelle (APCI) oder mittels XUV-Photonenenergie oder mittels der REMPI-Technik oder mittels einer Kombination der genannten Energiequellen oder Techniken ionisiert werden.

3. Verfahren gemäß Anspruch 2, **dadurch gekennzeichnet, dass** das Probengas mittels einer elektromagnetischen Energiequelle in einem λ-Bereich zwischen 100 nm und 500 nm ionisiert wird.

4. Verfahren gemäß einem der vorangehenden Ansprüche, **dadurch gekennzeichnet, dass** die Photodetachmentenergie mittels einer Laserenergiequelle oder anderen Lichtquellen eingebracht wird, worin der λ-Bereich zwischen 200 nm und 1400 nm liegt.

5. Verfahren gemäß einem der vorangehenden Ansprüche, **dadurch gekennzeichnet, dass** die Photodetachmentenergie mittels einer Laserenergiequelle gleichzeitig oder nacheinander bei zwei oder mehr verschiedenen Wellenlängen in λ-Bereichen zwischen 200 nm und 1400 nm eingebracht wird.

6. Verfahren gemäß einem der vorangehenden Ansprüche, **dadurch gekennzeichnet, dass** die Ionisierungsenergiequellen und/oder die Photodetachmentenergiequellen gepulst sind und dass die Pulse zeitverschoben sind.

7. Verfahren gemäß einem der vorangehenden Ansprüche, **dadurch gekennzeichnet, dass** die Photodetachmentquerschnitte der detektierten Ionen bestimmt und verglichen werden.

8. Verfahren gemäß einem der vorangehenden Ansprüche, **dadurch gekennzeichnet, dass** die Ionen, die zu einem Peak gehören, zusätzlich zur lonenmobilität durch den Photodetachmentquerschnitt charakterisiert werden

9. Verfahren gemäß einem der vorangehenden Ansprüche, **dadurch gekennzeichnet, dass** die Peakreinheit bestimmt wird durch die Bestimmung des Photodetachments von Ionen in den steigenden und fallenden Kanten des entsprechenden Peaks.

10. Vorrichtung(1) Zum Ausführen eines Verfahrens gemäß einem der vorangehenden Ansprüche, wobei die Vorrichtung (1) umfasst:
eine Driftröhre (2), die in einen Reaktionsbereich (4) und einen Driftbereich (5) getrennt ist;
einen Probeneinlass (6), der sich im Reaktionsbereich (4) befindet;
einen Driftgaseinlass (7), der sich im Driftbereich(5) befindet, eine Detektorplatte (3), die sich innerhalb des Driftbereichs (5)an einem Ende der Driftröhre (2) befindet; und
Mittels zum Bereitstellen von Ionisierungsenergie oder wenigstens einem Paar Fenster (8), die in der Driftröhre (2) innerhalb des Reaktionsbereichs (4) angeordnet sind;
**dadurch gekennzeichnet, dass**
wenigstens ein zusätzliches Paar Fenster (9) in der Driftröhre (2) innerhalb des Driftbereichs (5) der Driftröhre (2) vorhanden ist.

11. Vorrichtung gemäß Anspruch 10, **dadurch gekennzeichnet, dass** eines der Fenster (8) und/oder eines der Fenster (9) durch einen Glasfaseroptikeinlass ersetzt ist.

12. Vorrichtung gemäß Anspruch 10, **dadurch gekennzeichnet, dass** ein Paar Fenster (8) an gegenüberliegenden Enden eines Durchmessers der Driftröhre (2) in dem Reaktionsbereich (4) platziert ist, und dass es ein Paar zusätzlicher Fenster (9) gib, die an gegenüberliegenden Enden eines Durchmessers der Driftröhre (2) im Driftbereich (5) platziert sind, und dass die zusätzlichen Fenster (9) Glasfenster und/oder Quarzglasfenster sind.

13. Vorrichtung gemäß Anspruch 10, **dadurch gekennzeichnet, dass** der Reaktionsbereich (4) und der Driftbereich (5) voneinander getrennt sind durch wenigstens einen Verschluss oder ein Ionentor (10, 10a), wobei ein Verschluss (10) in der Nähe des Probeneinlasses (6) platziert ist, um den Reaktionsbereich zu begrenzen, und/oder ein Ionentor (10a) in Richtung stromaufwärts des Ionenstroms in der Nähe der zusätzlichen Fenster (9) platziert ist.

14. Verwendung eines Verfahrens gemäß irgendeinem der Ansprüche 1 bis für die Detektion von flüchtigen organischen Verbindungen, chemischen Kampfstoffen, illegalen Drogen, toxischen industriellen Verbindungen und Sprengstoffen.

15. Verwendung einer Vorrichtung gemäß irgendeinem der Ansprüche 10 bis 13 für die Detektion von flüchtigen organischen Verbindungen, chemischen Kampfstoffe, illegalen Drogen, toxischen industrieller Verbindungen und Sprengstoffen.

## Revendications

1. Méthode pour la détection d'anions en combinant la spectrométrie de mobilité ionique à la pression atmosphérique avec la spectroscopie de photodétachement, dans laquelle les étapes suivantes sont effectuées:
introduction d'un échantillon gazeux dans un tube de glissement;
ionisation des molécules de l'échantillon devant être détectées dans l'échantillon gazeux;
application d'un champ électrique sur les molécules ionisées de l'échantillon, par quel moyen les molécules ionisées de l'échantillon sont rendues mobiles dans le tube de glissement en direction d'un détecteur;
application d'énergie de photodétachement sur l'échantillon gazeux ionisé à proximité spatiale dudit détecteur, par quel moyen des électrons photodétachés sont engendrés;
détection simultanée des ions des molécules de l'échantillon et des électrons photodétachés ; et comparaison de l'intensité des pics des ions et des pics des électrons.

2. Méthode selon la revendication 1, **caractérisée en ce que** les molécules de l'échantillon sont ionisées au moyen d'une source d'énergie électromagnétique ou au moyen d'une source d'énergie laser ou au moyen d'une source d'énergie radioactive ou au moyen d'une source d'électronébulisation négative ou au moyen d'une source de décharge corona (APCI) ou par de l'énergie de photons XUV ou par la technique REMPI ou par une combinaison desdites sources d'énergie ou techniques.

3. Méthode selon la revendication 2, **caractérisée en ce que** l'échantillon gazeux est ionisé au moyen d'une source d'énergie électromagnétique dans une gamme de λ comprise entre 100 nm et 500 nm.

4. Méthode selon l'une des revendications précédentes, **caractérisée en ce que** l'énergie de photodétachement est appliquée au moyen d'une source d'énergie laser ou d'autres sources de lumière, dans laquelle la gamme de λ est comprise entre 200 nm et 1 400 nm.

5. Méthode selon l'une des revendications précédentes, **caractérisée en ce que** l'énergie de photodétachement est appliquée au moyen d'une source d'énergie laser simultanément ou consécutivement à deux longueurs d'onde différentes ou plus dans des gammes de λ comprises entre 200 nm et 1 400 nm.

6. Méthode selon l'une des revendications précédentes, **caractérisée en ce que** les sources d'énergie d'ionisation et/ou les sources d'énergie de photodétachement sont pulsées et **en ce que** les impulsions sont déplacées en temps opportun.

7. Méthode selon l'une des revendications précédentes, **caractérisée en ce que** les sections transversales de photodétachement des ions détectés sont déterminées et comparées.

8. Méthode selon l'une des revendications précédentes, **caractérisée en ce que** les ions appartenant à un pic sont caractérisés, en plus de la mobilité ionique, par la section transversale de photodétachement.

9. Méthode selon l'une des revendications précédentes, **caractérisée en ce que** la pureté de pic est déterminée par la détermination du photodétachement d'ions dans les fronts montant et descendant du pic respectif.

10. Dispositif (1) pour mettre en oeuvre une méthode selon l'une des revendications précédentes, par quel moyen le dispositif (1) comprend:
un tube de glissement (2), qui est divisé en une région de réaction (4) et une région de glissement (5);
une entrée d'échantillon (6), située dans la région de réaction (4);
une entrée de gaz de glissement (7), située dans la région de glissement (5);
une plaque de détecteur (3), située au sein de la région de glissement (5) à une extrémité du tube de glissement (2); et
des moyens pour fournir de l'énergie d'ionisation ou au moins une paire de fenêtres (8) qui sont placées dans le tube de glissement (2) au sein de la région de réaction (4);
**caractérisé en ce**
**qu'**au moins une paire supplémentaire de fenêtres (9) est présente dans le tube de glissement (2) au sein de la région de glissement (5) du tube de glissement (2).

11. Dispositif selon la revendication 10, **caractérisé en ce que** l'une des fenêtres (8) et/ou l'une des fenêtres (9) sont remplacées par une entrée de fibres de verre optiques.

12. Dispositif selon la revendication 10, **caractérisé en ce qu'**il y a une paire de fenêtres (8) placées sur des extrémités opposées d'un diamètre du tube de glissement (2) dans la région de réaction (4) et **en ce qu'**il y a une paire de fenêtres supplémentaires (9) placées sur des extrémités opposées d'un diamètre du tube de glissement (2) dans la région de glissement (5) et **en ce que** les fenêtres (8) et les fenêtres supplémentaires (9) sont des fenêtres en verre et/ou des fenêtres en verre de quartz.

13. Dispositif selon la revendication 10, **caractérisé en ce que** la région de réaction (4) et la région de glissement (5) sont séparées l'une de l'autre par au moins un obturateur ou une grille ionique (10, 10a), par quel moyen un obturateur (10) est placé à proximité de l'entrée d'échantillon (6) dans le but de délimiter la région de réaction et/ou une grille ionique (10a) est placée dans la direction en amont du flux d'ions à proximité des fenêtres supplémentaires (9).

14. Utilisation d'une méthode selon l'une quelconque des revendications 1 à 9 pour la détection de composés organiques volatils, d'agents de guerre chimiques, de drogues illicites, de composés industriels toxiques et d'explosifs.

15. Utilisation d'un dispositif selon l'une quelconque des revendications 10 à 13 pour la détection de composés organiques volatils, d'agents de guerre chimiques, de drogues illicites, de composés industriels toxiques et d'explosifs.
